# EUROPÄISCHE PATENTSCHRIFT

(11) **EP 1 023 013 B1**
(45) Veröffentlichungstag und Bekanntmachung des Hinweises auf die Patenterteilung: **21.05.2003**
(21) Anmeldenummer: 98954293.1
(22) Anmeldetag: 29.09.1998
(51) Int. Cl.: A61F 5/41

(54) **PENISEXTENSIONSGERÄT**
PENIS EXTENDER
DISPOSITIF D'ALLONGEMENT DU PENIS

(30) Priorität: 15.10.1997 DE 19745611
(43) Veröffentlichungstag der Anmeldung: 02.08.2000
(73) Patentinhaber: PHALOMED MANUFACTURING LIMITED, San Gwann SGN 09 (MT)
(72) Erfinder: Jochum, Herbert, 82541 Ammerland (DE)
(74) Vertreter: Köster, Hajo, Dr.
(86) Internationale Anmeldenummer: EP9806164
(87) Internationale Veröffentlichungsnummer: WO99018897

(56) Entgegenhaltungen:
- WO-A-96/26691
- WO-A-97/28764
- DE-U- 29 602 101
- FR-A- 1 605 238
- US-A- 4 440 183

## Beschreibung

Die vorliegende Erfindung betrifft ein Penisextensionsgerät zur Vergrößerung des männlichen Gliedes durch langzeitige Dehnungsbehandlung der Corpora cavernosa, umfassend ein Dehnelement, das sich zwischen einer lösbaren Befestigungseinrichtung zur Fixierung der Glans penis und einer als Widerlager dienenden Aufnahme für die Peniswurzel erstreckt.

Männern, die unter einem zu kleinen Penis leiden, wird gegenwärtig auf zwei Wegen Hilfe angeboten. Zum einen läßt sich der Penis operativ vergrößern; ein derartiger Eingriff ist jedoch extrem kostspielig und nicht ungefährlich. Ferner kommt eine langzeitige Dehnungsbehandlung der Corpora cavernosa unter Einsatz von Geräten der eingangs genannten Art in Betracht. Derartige Geräte sind beispielsweise den internationalen Patentanmeldungen WO96/26691 und WO97/28764 und dem deutschen Gebrauchsmuster 29602101 entnehmbar. Bei den bekannten Penisextensionsgeräten ist das Dehnelement übereinstimmend durch jeweils zwei Stangen gebildet, die etwa parallel zu dem zu streckenden Penis verlaufen. Diese Stangen stützen sich auf einem Ring bzw. einer Grundplatte, die den Penis im Bereich der Peniswurzel umgibt. Dabei ist die Länge der Stangen zwischen dem als Widerlager dienenden Ring einerseits und der Befestigungseinrichtung zur Fixierung der Glans penis andererseits einstellbar (WO96/26691 und WO97/28764), wobei ergänzend eine Vorspannung erzeugende Druckfedern vorgesehen sein können (WO97/28764); oder aber die Befestigungseinrichtung zur Fixierung der Glans penis ist auf den Stangen verschiebbar und an einer individuell gewünschten Stellung feststellbar (DE 29602101 U1).

Die bekannten gattungsgemäßen Penisextensionsgeräte weisen eine Mehrzahl gravierender Nachteile auf. Hervorzuheben ist vor allen Dingen, daß eine unauffällige Dehnungsbehandlung der Corpora cavernosa nicht möglich ist. Vielmehr steht das zu dehnende Glied während der Behandlung mittels der bekannten Streckmechanismen bis zu 90° vom Körper ab. Zudem ist der Streckmechanismus zwangsläufig länger als das zu dehnende Glied. Als Folge hiervon ist es praktisch unmöglich, während der Behandlung normale Kleidung zu tragen. Dies wiederum hat zur Folge, daß eine Anwendung der bekannten Penisextensionsgeräte praktisch nur zuhause bzw. in der privaten Sphäre in Betracht kommt. Dies resultiert wiederum in einer relativ geringen täglichen Anwendungsdauer, die sich in einem für die betroffenen Männer unbefriedigenden Erfolg niederschlägt.

Aus den vorstehend dargelegten Nachteilen bekannter gattungsgemäßer Penisextensionsgeräte leitet sich die der vorliegenden Erfindung zugrundeliegende Aufgabenstellung ab, die darin besteht, ein Penisextensionsgerät der eingangs genannten Art zu schaffen, das unauffällig am Körper getragen werden kann.

Gemäß der vorliegenden Erfindung wird diese Aufgabe dadurch gelöst, daß das Dehnelement als flexibles, um den Körper des Benutzers schlingbares Band ausgebildet ist. Die erfindungsgemäße Gestaltung des Dehnelements hat zur Folge, daß der zu dehnende Penis während der Dehnungsbehandlung etwa seitwärts gerichtet eng am Körper des Benutzers des erfindungsgemäßen Geräts anliegt. Dies ermöglicht eine Dehnungsbehandlung unter normaler Kleidung, weshalb die Behandlung nicht auf Zeiten, in denen sich der Betroffene in der privaten Sphäre aufhält, beschränkt zu werden braucht; vielmehr kann die Anwendung unauffällig während der täglichen beruflichen Beschäftigung über Stunden erfolgen. Als Folge hiervon tritt bei einer konsequenten Anwendung des erfindungsgemäßen Geräts eine dauerhafte Dehnung des Penis bereits nach wenigen Monaten ein. Die physische und psychische Wirkung einer solchen Vergrößerung auf den Betroffenen ist enorm.

Ergänzend zu den vorstehend dargelegten Vorteilen zeichnet sich das erfindungsgemäße Penisextensionsgerät im Vergleich zum gattungsgemäßen Stand der Technik durch einen besonders geringen apparativen Aufwand aus, was sich in entsprechend geringen Herstellkosten niederschlägt.

Eine erste bevorzugte Weiterbildung der Erfindung zeichnet sich dadurch aus, daß das Band elastisch dehnbar ist. In diesem Sinne kann das Band insbesondere als Gummiband ausgeführt sein. Die elastische Dehnbarkeit des Bandes führt zu einem besonders wirksamen konstanten Zug, der das zu streckende Glied kontinuierlich dehnt und somit dessen allmähliche Vergrößerung begünstigt.

Eine weitere bevorzugte Weiterbildung der Erfindung ist dadurch gekennzeichnet, daß das Band längenverstellbar ist. Durch eine Verkürzung oder Verlängerung des Bandes kann das erfindungsgemäße Penisextensionsgerät an den Körperumfang seines Benutzers angepaßt werden. Zudem läßt sich auf diese Weise die Kraft, mit der das Glied gestreckt wird, dosiert einstellen.

Eine wiederum andere bevorzugte Weiterbildung der Erfindung zeichnet sich dadurch aus, daß die Aufnahme für die Peniswurzel als Ringmanschette ausgebildet ist. Diese Ringmanschette, an der das Band im Bereich seines einen Endes festgelegt sein kann, wird zu Beginn jeder Dehnungsbehandlung über den Penis bis zu dessen Wurzel geschoben. Danach wird das Band um den Körper des Benutzers gelegt und die Glans penis an der Befestigungseinrichtung fixiert. Die Ringmanschette leitet die Gegenkraft zu der auf die Glans penis ausgeübte Zugkraft in die Peniswurzel ein, so daß ein in sich geschlossenes Kraftsystem vorliegt.

Die Befestigungseinrichtung für die Glans penis kann bevorzugt einen schalenförmigen Objektträger umfassen. Dabei kann insbesondere ein schlaufenförmiges Bändchen vorgesehen sein, mit welchem die Glans penis am Objektträger fixierbar ist.

Eine wiederum andere Weiterbildung des erfindungsgemäßen Penisextensionsgeräts zeichnet sich dadurch aus, daß eine Halteelement vorgesehen ist, das sich zwischen der Aufnahme für die Peniswurzel und/oder dem dieser zugeordneten Ende des Bandes einerseits und der Befestigungseinrichtung für die Glans penis und/oder dem dieser zugeordneten Ende des Bandes andererseits erstreckt. Dieses Halteelement, das beispielsweise als nichtdehnbares Band ausgeführt sein kann, ist während der bestimmungsgemäßen Verwendung des Penisextensionsgeräts entspannt. Es tritt in Funktion, wenn der Benutzer die Fixierung der Glans penis an der Befestigungseinrichtung löst, beispielsweise um Wasser zu lassen. Das Halteelement verhindert in diesem Falle, daß die Befestigungseinrichtung unter dem Zug des elastisch dehnbaren Bandes auf den Rücken des Benutzers schnellt. Das Halteelement ist dabei so bemessen, daß es die von der Glans penis gelöste Befestigungseinrichtung auf der Seite der Hüfte des Benutzers hält, so daß eine erneute Fixierung der Glans penis problemlos möglich ist. Zudem verhindert das Halteelement, daß das Penisextensionsgerät nach unten rutscht, wenn sein Benutzer es sozusagen in "Bereitschaft" trägt, ohne gerade eine Dehnungsbehandlung durchzuführen.

Im folgenden wird die vorliegende Erfindung anhand zweier in der Zeichnung dargestellter bevorzugter Ausführungsbeispiele näher erläutert. Dabei zeigt
- Fig. 1: eine Gesamtansicht einer ersten bevorzugten Ausführungsform des erfindungsgemäßen Penisextensionsgeräts während seiner bestimmungsgemäßen Verwendung; und
- Fig. 2: veranschaulicht eine besonders bevorzugte Gestaltung der Befestigungseinrichtung zur Fixierung der Glans penis.

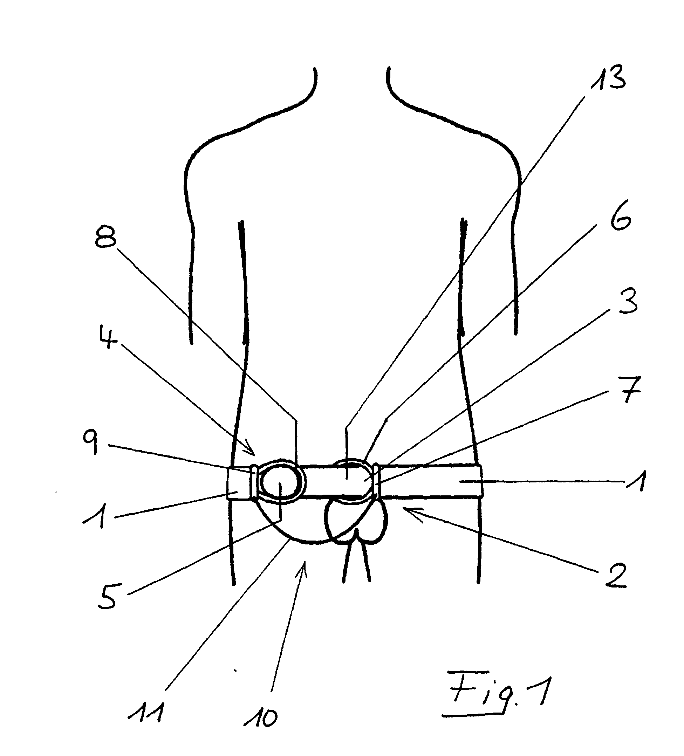

Das in Fig. 1 veranschaulichte Penisextensionsgerät umfaßt ein elastisch dehnbares, flaches Band 1, an dessen einem Ende eine Aufnahme 2 für die Peniswurzel 3 und an dessen anderem Ende eine Befestigungseinrichtung 4 zur Fixierung der Glans penis 5 befestigt ist. Die Aufnahme 2 für die Peniswurzel 3 ist dabei als Ringmanschette 6 ausgebildet, wobei die Verbindung mit dem Band 1 über einen sich über die gesamte Breite des Bandes erstreckenden Klemmsteg 7 hergestellt ist. Die Befestigungseinrichtung 4 zur Fixierung der Glans penis 5 umfaßt ein schlaufenförmiges Bändchen 8, dessen beide Enden an einem Klemmsteg 9 festgelegt sind, der seinerseits an dem zweiten Ende des Bandes 1 befestigt ist. Zwischen den beiden Klemmstegen 7 und 9 erstreckt sich ein Halteelement 10 in Form einer Schnur 11, die während der bestimmungsgemäßen Verwendung des Penisextensionsgeräts lose herabhängt und erst zum Tragen kommt, wenn die Befestigungseinrichtung 4 von der Glans penis 5 gelöst wird. Die Befestigung der Schnur 11 ist an einem der beiden Klemmstege 7 bzw. 9 lösbar; die Verbindung wird an dieser Stelle erst hergestellt, wenn das Band 1 um den Körper des Benutzers herumgelegt worden ist.

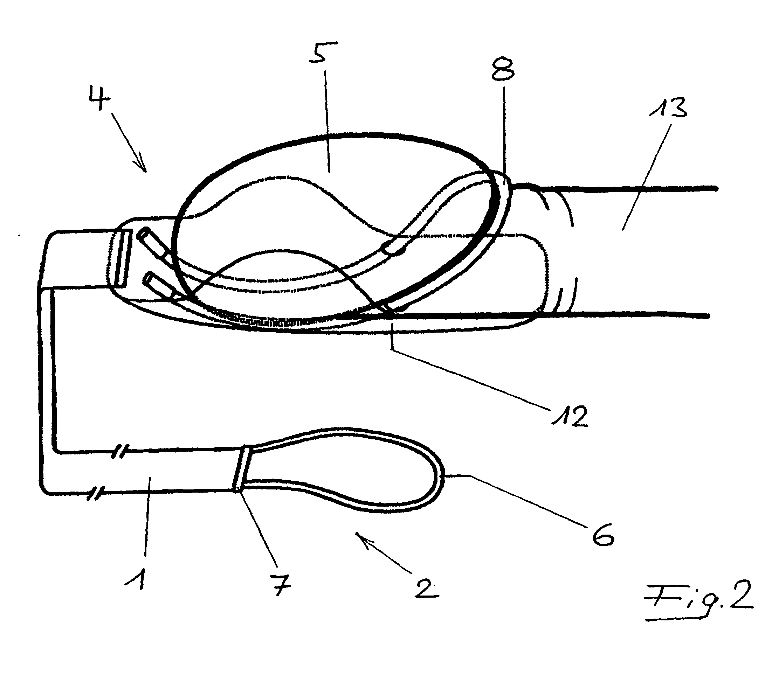

Fig. 2 veranschaulicht in perspektivischer Darstellung, daß die der Fixierung der Glans penis 5 dienende Befestigungseinrichtung 4 einen schalenförmigen Objektträger 12 umfassen kann, der die Glans penis 5 und das vordere Ende des Schaftes 13 von unten her teilweise umgibt. Der Objektträger 12 ist mit einem Ende des Bandes 1 fest verbunden. An ihm sind die beiden Enden des Bändchens 8, mit welchem die Glans penis an dem Objektträger fixiert wird, festgelegt.

## Patentansprüche

1. Penisextensionsgerät zur Vergrößerung des männlichen Gliedes durch langzeitige Dehnungsbehandlung der Corpora cavernosa, umfassend ein Dehnelement, das sich zwischen einer Befestigungseinrichtung (4) zur Fixierung der Glans penis (5) und einer als Widerlager dienenden Aufnahme (2) für die Peniswurzel (3) erstreckt,
**dadurch gekennzeichnet,**
**daß** das Dehnelement als flexibles, um den Körper des Benutzers schlingbares Band (1) ausgebildet ist.

2. Penisextensionsgerät nach Anspruch 1,
**dadurch gekennzeichnet,**
**daß** das Band (1) elastisch dehnbar ist.

3. Penisextensionsgerät nach Anspruch 1 oder Anspruch 2,
**dadurch gekennzeichnet,**
**daß** das Band (1) längenverstellbar ist.

4. Penisextensionsgerät nach einem der Ansprüche 1 bis 3,
**dadurch gekennzeichnet,**
**daß** die Aufnahme (2) für die Peniswurzel (3) als Ringmanschette (6) ausgebildet ist.

5. Penisextensionsgerät nach einem der Ansprüche 1 bis 4,
**dadurch gekennzeichnet,**
**daß** die Befestigungseinrichtung (4) für die Glans penis (5) einen Objektträger (12) umfaßt.

6. Penisextensionsgerät nach Anspruch 5,
**dadurch gekennzeichnet,**
**daß** die Befestigungseinrichtung (4) des weiteren ein Bändchen (8) umfaßt, mit welchem die Glans penis (5) am Objektträger (12) fixierbar ist.

7. Penisextensionsgerät nach einem der Ansprüche 5 oder 6,
**dadurch gekennzeichnet,**
**daß** der Objektträger (12) schalenförmig ausgebildet ist.

8. Penisextensionsgerät nach einem der Ansprüche 1 bis 7,
**dadurch gekennzeichnet,**
**daß** ein Halteelement (10) vorgesehen ist, das sich zwischen der Aufnahme (2) für die Peniswurzel (3) und/oder dem dieser zugeordneten Ende des Bandes (1) einerseits und der Befestigungseinrichtung (4) für die Glans penis (5) und/oder dem dieser zugeordneten Ende des Bandes (1) andererseits erstreckt.

## Claims

1. Penis extension device for enlarging the male penis by prolonged stretching of the corpora cavernosa, consisting of an expansion element located between a fixing device (4) for the purpose of holding the glans penis (5) and a retainer (2) designed as a countersupport for the root of the penis (3),
**characterized in that**
the expansion element is in the form of a flexible belt (1) to be wrapped around the user's waist.

2. Penis extension device as set forth in claim 1,
**characterized in that**
the belt (1) can be stretched elastically.

3. Penis extension device as set forth in claims 1 and 2,
**characterized in that**
the belt (1) can be adjusted in length.

4. Penis extension device as set forth in any of claims 1 to 3,
**characterized in that**
the retainer (2) for the root of the penis (3) is in the shape of a ring collar (6).

5. Penis extension device as set forth in any of claims 1 to 4,
**characterized in that**
the fixing device (4) for the glans penis (5) incorporates a receptacle (12).

6. Penis extension device as set forth in claim 5,
**characterized in that**
the fixing device (4) additionally incorporates a band (8) with which the glans penis (5) is secured to the receptacle (12).

7. Penis extension device as set forth in either of claims 5 or 6,
**characterized in that**
the receptacle (12) is cup-shaped.

8. Penis extension device as set forth in any of claims 1 to 7,
**characterized in that**
a holding element (10) is located between the retainer (2) for the root of the penis (3) and/or the appropriate end of the belt (1) on the one side and the fixing device (4) for the glans penis (5) and/or the appropriate end of the belt (1) on the other.

## Revendications

1. Dispositif d'extension du pénis destiné à développer le membre viril par une extension prolongée du corps caverneux, comportant un élément extenseur placé entre un dispositif d'attache (4) du gland (5) et une fixation (2), servant de contre-appui, pour la racine du pénis (3), **caractérisé par**
**le fait, que** l'élément extenseur est conçu sous forme de sangle (1) souple à nouer autour du corps de l'utilisateur.

2. Dispositif d'extension du pénis suivant la revendication 1,
**caractérisé par**
**le fait que** la sangle (1) est élastique.

3. Dispositif d'extension du pénis suivant la revendication 1 ou la revendication 2, **caractérisé par**
**le fait que** la longueur de la sangle (1) est réglable.

4. Dispositif d'extension du pénis suivant l'une des revendications 1 à 3, **caractérisé par**
**le fait que** la fixation (2) pour la racine du pénis (3) est conçue sous forme d'anneau (6).

5. Dispositif d'extension du pénis suivant l'une des revendications 1 à 4,
**caractérisé par**
**le fait que** le dispositif d'attache (4) du gland (5) comporte un support (12).

6. Dispositif d'extension du pénis suivant la revendication 5,
**caractérisé par**
**le fait que** le dispositif d'attache (4) comporte en outre un cordon (8) permettant de fixer le gland (5) au support (12).

7. Dispositif d'extension du pénis suivant l'une des revendications 5
ou 6, **caractérisé par**
**le fait que** le support (12) a une forme creuse.

8. Dispositif d'extension du pénis suivant l'une des revendications 1 à 7,
**caractérisé par**
**le fait qu'**il est prévu un élément de retenue (10) qui est placé entre la fixation (2) pour la racine du pénis (3) et/ou l'extrémité correspondante de la sangle (1) d'une part et le dispositif d'attache (4) du gland (5) et/ou l'extrémité correspondante de la sangle (1) d'autre part.
